(19) **Europäisches Patentamt / European Patent Office / Office européen des brevets**

(11) **EP 3 562 467 B2**

(12) # NEW EUROPEAN PATENT SPECIFICATION
## After opposition procedure

(45) Date of publication and mention
of the opposition decision:
**06.09.2023 Bulletin 2023/36**

(45) Mention of the grant of the patent:
**01.04.2020 Bulletin 2020/14**

(21) Application number: **17816519.7**

(22) Date of filing: **27.11.2017**

(51) International Patent Classification (IPC):
*A61K 8/368* (2006.01)      *A61K 8/81* (2006.01)
*A61Q 19/10* (2006.01)      *A61K 8/36* (2006.01)
*A61K 8/41* (2006.01)      *A61Q 17/00* (2006.01)

(52) Cooperative Patent Classification (CPC):
**A61Q 19/10; A61K 8/361; A61K 8/368;
A61K 8/416; A61K 8/817; A61Q 17/005**

(86) International application number:
**PCT/EP2017/080507**

(87) International publication number:
**WO 2018/121946 (05.07.2018 Gazette 2018/27)**

(54) **AN ANTIMICROBIAL COMPOSITION**

ANTIMIKROBIELLE ZUSAMMENSETZUNG

COMPOSITION ANTIMICROBIENNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.12.2016 EP 16206935**

(43) Date of publication of application:
**06.11.2019 Bulletin 2019/45**

(73) Proprietors:
• **Unilever Global IP Limited
Wirral, Merseyside CH62 4ZD (GB)**
Designated Contracting States:
**CY DE GB IE IT MT**
• **Unilever IP Holdings B.V.
3013 AL Rotterdam (NL)**
Designated Contracting States:
**AL AT BE BG CH CZ DK EE ES FI FR GR HR HU
IS LI LT LU LV MC MK NL NO PL PT RO RS SE SI
SK SM TR**

(72) Inventors:
• **BARNE, Sameer Keshav
Bangalore 560066 (IN)**
• **SAJI, Maya Treesa
Bangalore 560066 (IN)**

(74) Representative: **van den Brom, Coenraad Richard
Unilever Patent Group
Bronland 14
6708 WH Wageningen (NL)**

(56) References cited:
WO-A1-00/61107      WO-A1-01/97799
WO-A1-98/55095      WO-A1-98/55099
WO-A1-99/24012      WO-A1-2016/050493
WO-A2-01/28338      CN-A- 105 769 635
JP-A- H08 143 899      JP-A- 2014 176 801
RU-C1- 2 456 022      US-A- 6 113 933
US-A1- 2003 089 891

• **Wikipedia Extract ?Propionsäure?**
• **Wikipedia Extract ?Hexansäure?**
• **Chemical-Abstracts' SciFinder®, Dodecanoic
acid**
• **Wikipedia Extract .Succinsäure
(Bernsteinsäure)"**
• **Experimental Report of September 2021**
• **Experimental Report of September 2022**

EP 3 562 467 B2

## Description

## Technical Field

The present invention relates to an antimicrobial composition and more particularly to an antimicrobial composition at the pH of skin.

## Background of the invention

People try to take good care of the external surface of their bodies as well as those of their pets to enable good overall health. Specific skin related issues that people care about include, good skin health free of infections, good skin tone and adequate moisturisation. Oral cavity is another external surface that people take active care to maintain. They prefer their oral cavity including the gums and teeth to be free of problems like cavities, tartar, gingivitis, caries, and bad breath, also known as halitosis, and plaque. Hair and scalp care are also of concern to people. People generally prefer to have thick long hair with minimum hair fall. Dandruff is a commonly occurring scalp problem for which a fungal microorganism has been implicated.

All of the above surfaces including skin, oral cavity and scalp hygiene are generally achieved by keeping them free of infections. One way to tackle infections is to treat them with antimicrobials after the infection has set in. Another approach is to leave a minimal amount of antimicrobial active on the surface so that any invading microorganism is killed or inactivated so as to minimize spread of disease. Some of the bacteria like *E.coli, S aureus* are generally exists on the skin. These bacteria does not have any pathogenic effect per se on the skin. However when this goes inside the body through ingestion, they induces their pathogenic effect. Therefore keeping the external surface of the body e.g. hand, scalp free of this bacteria helps in achieving the desired hygiene.

Various routes for improving the biocidal activity of soap based cleansing compositions have been suggested.

US2008014247A (Lu et al., 2008) discloses a composition having metal containing material, stearic acid and a pharmaceutically acceptable carrier to treat conditions caused by gram-positive, gram-negative, fungal pathogens and/or antibiotic-resistant bacteria. It further provides a method for inhibiting biofilm proliferation. The metal containing material can be silver.

US6051614A discloses a method for preparing a non-aqueous dispersion of particles of a metal and/or a metal compound, which comprises contacting an aqueous dispersion of particles of a metal and/or a metal compound with a water-immiscible, non-aqueous liquid in the presence of a surfactant and in the presence or absence of a water-soluble inorganic acid salt and/or a water-soluble organic acid salt exhibiting substantially no surface activity, wherein when the contacting of the aqueous dispersion with the non-aqueous liquid is conducted in the absence of the salt, the salt is added after the contacting, thereby causing the particles to be migrated from the aqueous dispersion into the non-aqueous liquid.

US3050467 B1 (Horowitz et al. 1962) discloses an antimicrobial cleansing composition consisting essentially of a mixture of a water-soluble soap and a silver salt of partially depolymerized alginic acid. The composition provides synergestic antimicrobial activity.

US2011224120 AA (Henkel) discloses liquid washing compositions having surfactant, silver and/or a silver compound and a non-neutralized fatty acid.

However, addition of soap invariably increases the pH of the composition. High pH composition is harsh on the skin. Consumer also has a preference to use a formulation with pH close to that of skin.

WO 01/28338 (The Procter & gamble Company, 2001) discloses antimicrobial compositions comprising: (a) a safe and effective amount of a benzoic acid analog; (b) a safe and effective amount of a metal salt; and (c) a dermatologically acceptable carrier for the acid and salt wherein said composition has a pH of from about 1 to about 7.

Another problem is most of the soap based antimicrobial compositions has to be applied for sufficiently long time to get the antimicrobial benefit. However most of the people especially kids do not wash their hand/skin for long enough.

Hence, there is a need to provide an antimicrobial composition at skin pH with the ability to provide the desired antimicrobial benefits at relatively short contact time.

It is therefore an object of the present invention to provide an antimicrobial composition.

It is another object of the present invention to provide an antimicrobial composition at the pH close to that of human skin.

It is another object of the present to provide an antimicrobial composition with the ability to provide the antimicrobial benefit at relatively short contact time.

The present inventors have surprisingly found that a combination of an ammonium salt having at least one covalently bonded hydrogen attached to the nitrogen or a surfactant with ammonium group as counter ion when combined with specific carboxylic acids provides a synergistic antibacterial activity in relatively short contact times.

## Summary of the invention

In a first aspect, the present invention provides an antimicrobial composition comprising:

  a) 1 to 40% by weight of an ammonium salt having at least one covalently bonded hydrogen attached to the nitrogen or of an anionic surfactant with ammonium group as counter ion or mixtures thereof; and,

b) 0.1 to 20% by weight of a carboxylic acid with $pK_a$ value is in between 4.5 to 6.5 and wherein the carboxylic acid is selected from fatty acid having chain length $C_4$ to $C_{16}$, Cyclic aliphatic carboxylic acid, aromatic carboxylic acid or mixtures thereof;

wherein the pH of the composition is in the range of 4.5 to 6.5;
and wherein the total amount of carboxylic acid with $pK_a$ value greater than 4.5 is from 0.1 to 20% by weight.

In a second aspect, the present invention provides a method of cleaning or disinfecting a surface comprising the step of applying a composition of the first aspect on to said surface.

Any feature of one aspect of the present invention may be utilized in any other aspect of the invention. The word "comprising" is intended to mean "including" but not necessarily "consisting of" or "composed of." In other words, the listed steps or options need not be exhaustive. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

**Detailed description of the invention**

The present invention provides an antimicrobial composition comprising:

a) 1 to 40% by weight of an ammonium salt having at least one covalently bonded hydrogen attached to the nitrogen or of an anionic surfactant with ammonium group as counter ion or mixtures thereof; and,
b) 0.1 to 20% by weight of a carboxylic acid with $pK_a$ value is in between 4.5 to 6.5 and wherein the carboxylic acid is selected from fatty acid having chain length $C_4$ to $C_{16}$, Cyclic aliphatic carboxylic acid, aromatic carboxylic acid or mixtures thereof;

wherein the pH of the composition is in the range of 4.5 to 6.5;
and wherein the total amount of carboxylic acid with $pK_a$ value greater than 4.5 is from 0.1 to 20% by weight.

Antimicrobial composition as mentioned herein above preferably means any composition, which is capable of killing or at least cause substantial reduction of the common disease causing microbes. The common disease causing gram-positive organisms includes *Staphylococcus, Streptococcus* and *Enterococcus* spp. Some of common disease causing gram-negative organisms includes *Escherichia coli, Salmonella, Klebsiella* and *Shigella. Escherichia coli and Salmonella* can cause severe gastrointestinal illnesses.

The composition of the present invention comprises 1 to 40%, preferably 2 to 35%, more preferably 2 to 25%, further more preferably 2 to 20% and most preferably 5 to 20% by weight of an ammonium salt having at least one covalently bonded hydrogen attached to the nitrogen or an anionic surfactant with ammonium group as counter ion or mixtures thereof.

Ammonium salt:

The composition of the present invention comprises an ammonium salt having at least one covalently bonded hydrogen attached to the nitrogen. A covalent bond, also called a molecular bond, is a chemical bond that involves the sharing of electron pairs between atoms. These electron pairs are known as shared pairs or bonding pairs, and the stable balance of attractive and repulsive forces between atoms, when they share electrons, is known as covalent bonding. Covalent bonding includes many kinds of interactions, including $\sigma$ - bonding, $\pi$ - bonding, metal-to-metal bonding, agostic interactions, bent bonds, and three-center two-electron bonds.

The ammonium salt in case of composition of the present invention having at least one covalently bonded hydrogen attached to the nitrogen.

The preferred ammonium salts are selected from ammonium chloride, ammonium benzoate, ammonium citrate, ammonium carbonate, ammonium acetate, ammonium sulphate, isopropyl ammonium chloride or mixtures thereof.

An anionic surfactant with ammonium group as counter ion

When the hydrophilic part of the surfactant consists of a negatively charged group like a sulphonate, sulphate or carboxylate the surfactant is called anionic surfactant. These acts as an active surface agent to lower the surface tension of liquids. This allows them to bind to impurities and particles that are suspended in the liquid, which makes them effective cleaning agents in water. In small concentrations, they can also cause the foaming of compounds in water by creating large numbers of small bubbles of gas, and this makes them effective in cosmetics such as shampoo, toothpaste, hand wash and body wash products.

The composition of the present invention comprises an anionic surfactant with ammonium as counter ion. Examples of anionic surfactants with ammonium as counter ion suitable for use herein include, but are not limited to, ammonium lauryl sulphate, ammonium lauryl sulfate, ammonium laureth sulfate, ammonium dodecyl benzene sulfonate, or a combination thereof.

Carboxylic Acid

The composition of the present invention also comprises 0.1 to 20%, preferably 0.1 to 15%, more preferably 0.2 to 10% and most preferably 0.2 to 5% by weight of a carboxylic acid with $pK_a$ value greater than 4.5.

Carboxylic acids are organic acids having (-COOH) group as a part of their structure. An acid dissociation constant ($K_a$) represents the strength of an acid in a solution. The negative logarithm of $K_a$ is represented by pKa, which is quite relevant for practical applications of $K_a$.

$$pK_a = - \log_{10} K_a$$

The higher the value of $pK_a$, the lower the extent of dissociation at a given pH. This is governed by the well-known Henderson-Hasselbalch equation.

According to the present invention the carboxylic acid having $pK_a$ value greater than 4.5, more preferably greater than 4.6 and most preferably greater than 4.7.

The range of $pK_a$ is in between 4.5 to 6.5 and more preferably 4.6 to 6.

Preferably, the carboxylic acid is selected from fatty acid having chain length C4 to C16, more preferably C6 to C12 and most preferably from C6 to C10. The carboxylic acids are preferably selected from linear or branched aliphatic carboxylic acids, cyclic aliphatic carboxylic acids and mixtures thereof. Substituted Carboxylic acid with the above defined pKa range may also be preferably used. The most preferred substitution are methyl and/or ethyl. The most preferred aliphatic carboxylic acids are selected from hexanoic acid (pKa: 4.88), cyclohexanoic acid (pKa:4.91), 2-ethyl hexanoic acid (pKa: 4.72), octanoic acid (pKa: 4.89), 4-methyl octanoic acid (pKa: 4.93) and mixtures thereof.

One of the objective of the present invention is to provide a composition at skin pH. Therefore, pH of the composition of the present invention is in the range of 4.5 to 6.5, more preferably 4.5 to 6 and most preferably about 5.

The composition of the present invention is a synergistic antimicrobial composition. The synergy effects is observed by combining 1 to 40% by weight of an ammonium salt having at least one covalently bonded hydrogen attached to the nitrogen or an anionic surfactant with ammonium group as counter ion or mixtures thereof and 0.1 to 20% by weight of a carboxylic acid with $pK_a$ value in between 4.5 to 6.5 and wherein the carboxylic acid is selected from fatty acid having chain length $C_4$ to $C_{16}$, cyclic aliphatic carboxylic acid, aromatic carboxylic acid or mixtures thereof. The synergistic antimicrobial composition of the present invention in the concentration range as mentioned above found to be effective against both gram-positive and gram-negative organisms.

The synergistic antimicrobial composition of the present invention also comprises a cosmetically acceptable base. The base formulation may be varied depends on the kinds of application.

The composition of the present invention preferably in the form of a leave-on composition. The leave-on composition may be in the form of vanishing cream or may be in the form of a sanitizer composition. The most preferred application being the hand sanitization.

The cosmetically acceptable base is preferably a cream, lotion, gel or emulsion.

Personal care compositions (leave-on) may be prepared using different cosmetically acceptable emulsifying or non-emulsifying systems and vehicles. A highly suitable base is a cream. Vanishing creams are especially preferred. Vanishing cream bases generally comprise 5 to 25% fatty acid and 0.1 to 10% soap. Vanishing cream base gives a highly appreciated matty feel to the skin. C12 to C20 fatty acids are especially preferred in vanishing cream bases, further more preferred being C14 to C18 fatty acids. The most preferred fatty acid is stearic acid. The fatty acid in the composition is more preferably present in an amount in the range of 5 to 20% by weight of the composition. Soaps in the vanishing cream base include alkali metal salt of fatty acids, like sodium or potassium salts, most preferred being potassium stearate. The soap in the vanishing cream base is generally present in an amount in the range of 0.1 to 10%, more preferably 0.1 to 3% by weight of the composition. Generally, the vanishing cream base in personal care compositions is prepared by taking a desired amount of total fatty matter and mixing with potassium hydroxide in desired amounts. The soap is usually formed insitu during the mixing.

An especially suitable cosmetically acceptable base is one which comprises a water-in-oil emulsion comprising silicone oils as the continuous phase. The water in oil emulsions preferably comprise a cross-linked silicone elastomer blend.

Inclusion of silicone elastomer blend in a water-in-oil emulsion may be used as the cosmetically acceptable base for preparing the compositions of the present invention. While silicone fluids may be used, silicone elastomers which are cross-linked, are especially preferred. The creation of cross-linkages between linear polymers, such as dimethicone, converts the linear polymer into a silicone elastomer. In contrast to silicone fluid polymers, the physical properties of elastomers are typically dependent on the number of cross-linkages, rather than molecular weight. The ability of silicone elastomers to swell makes them ideal thickeners for oil phases. The elastomers have a very smooth and soft feel when applied to skin or hair. They can also be used as delivery agents for fragrances, vitamins and other additives in cosmetic compositions.

Suitable silicone elastomer blends or gels which are commercially available and suitable for inclusion in the composition of the invention and found to provide the enhanced stability are: Dow Corning® EL-8051 IN Silicone Organic Elastomer Blend [INCI Name: Isodecyl Neopentanoate (and) Dimethicone/Bis Isobutyl PPG-20 Crosspolymer]; EL-8050 [INCI Name: Isododecane (and) Dimethicone/Bis-Isobutyl PPG 20 Crosspolymer] DC 9040, DC9041, DC9045 (Dimethicone crosspolymer); DC 9506, 9509 (Dimethicone vinyl dimethicone crosspolymer); Shin-Etsu KSG-15, KSG-16, KSG-17 (Dimethicone vinyl dimethicone crosspolymer). It is further preferred that the composition comprises 5 to 50% silicone elastomer by weight of the composition.

In the case of wash-off composition, In addition to the essential ingredients as described earlier, preferred embodiments of the cleansing compositions may also include other optional and preferred ingredients for their known benefits. The type and content will largely depend on the nature and type of cleansing composition as well as general principles of formulation science.

Where the composition is in the form of a bar of soap or a liquid soap, it is preferred that the composition contains free fatty acids. Preferred embodiments contain 0.01 wt% to 10 wt% free fatty acid, especially when major portion of the surfactant is soap based. Potentially suitable fatty acids are C8 to C22 fatty acids. Preferred fatty acids are C12 to C18, preferably predominantly saturated, straight-chain fatty acids. However, some unsaturated fatty acids can also be employed. Of course the free fatty acids can be mixtures of shorter chain length (e.g., C10 to C14) and longer chainlength (e.g., C16-C18) chain fatty acids. For example, one useful fatty acid is fatty acid derived from high-laurics triglycerides such as coconut oil, palm kernel oil, and babasu oil. The fatty acid can be incorporated directly or they can be generated in-situ by the addition of a protic acid to the soap during processing. Examples of suitable protic acids include: mineral acids such as hydrochloric acid and sulfuric acid, adipic acid, citric acid, glycolic acid, acetic acid, formic acid, fumaric acid, lactic acid, malic acid, maleic acid, succinic acid, tartaric acid and polyacrylic acid. However, care should be taken that the residual electrolyte in the bar does not substantially reduce the effectiveness of the anticracking agent. The level of fatty acid having a chain length of 14 carbon atoms and below should generally not exceed 5.0%, preferably not exceed about 1 % and most preferably be 0.8% or less based on the total weight of the continuous phase.

Water soluble/dispersible polymers is an optional ingredient that is highly preferred to be included in composition. These polymers can be cationic, anionic, amphoteric or nonionic types with molecular weights higher than 100,000 Dalton. They are known to increase the viscosity and stability of liquid cleanser compositions, to enhance in-use and after-use skin sensory feels, and to enhance lather creaminess and lather stability. Amount of the polymers, when present, may range from 0.1 to 10% by weight of the composition.

Examples of water soluble/or dispersible polymers include the carbohydrate gums such as cellulose gum, microcrystalline cellulose, cellulose gel, hydroxyethyl cellulose, hydroxypropyl cellulose, sodium carboxymethylcellulose, methyl cellulose, ethyl cellulose, guar gum, gum karaya, gum tragacanth, gum arabic, gum acacia, gum agar, xanthan gum and mixtures thereof; modified and nonmodified starch granules and pregelatinized cold water soluble starch; emulsion polymers such as Aculyn® 28, Aculyn® 22 or Carbopol® Aqua SF1; cationic polymer such as modified polysaccharides including cationic guar available from Rhone Poulenc under the trade name Jaguar® C13S, Jaguar® C14S, Jaguar® C17, or Jaguar® C16; cationic modified cellulose such as UCARE® Polymer JR 30 or JR 40 from Amerchol; N-Hance® 3000, N-Hance® 3196, N-Hance® GPX 215 or N-Hance® GPX 196 from Hercules; synthetic cationic polymer such as Merquat® 100, Merquat® 280, Merquat® 281 and Merquat® 550 sold by Nalco; cationic starches such as StaLok® 100, 200, 300 and 400 sold by Staley Inc.; cationic galactomannans such as Galactasol® 800 series by Henkel, Inc.; Quadrosoft® LM-200; and Polyquaternium-24®. Also suitable are high molecular weight polyethylene glycols such as Polyox® WSR-205 (PEG 14M), Polyox® WSR-N-60K (PEG 45), and Polyox® WSR-301 (PEG 90M).

The composition of the invention may additionally comprise a skin-lightening agent. Apart from niacinamide which is anyway presence as one of the essential component of the present compostion, other well known skin lightening agents e.g. aloe extract, ammonium lactate, arbutin, azelaic acid, kojic acid, butyl hydroxy anisole, butyl hydroxy toluene, citrate esters, 3 diphenyl propane derivatives, 2, 5 dihydroxybenzoic acid and its derivatives, ellagic acid, fennel extract, gluco pyranosyl-1-ascorbate, gluconic acid, glycolic acid, green tea extract, hydroquinone, 4 hydroxyanisole and its derivatives, 4-hydroxy benzoic acid derivatives, hydroxycaprylic acid, lemon extract, linoleic acid, magnesium ascorbyl phosphate, mulberry root extract, 2,4 resorcinol derivatives, 3,5 resorcinol derivatives, salicylic acid, vitamins like vitamin B6, vitamin B12, vitamin C, vitamin A, a dicarboxylic acid, resorcinol derivatives, hydroxycarboxylic acid like lactic acid and their salts e.g. sodium lactate, and mixtures thereof.

Preferably, the composition may have sunscreen. Any sunscreen that can be suitably used with the base may be added. Both, UVA and UVB sunscreens may preferably be added.

The composition of the invention preferably comprises a UV- A sunscreen which is a dibenzoylmethane or its derivatives. Preferred dibenzoylmethane derivatives are selected from 4-tert-butyl-4'-methoxydibenzoylmethane, 2-methyldibenzoylmethane, 4-methyldibenzoylmethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxydibenzoyl methane, 2,4-dimethyl-4'- methoxy dibenzoylmethane or 2,6-dimethyl-4-tert-butyl-4'-methoxydibenzoylmethane. The most preferred dibenzoylmethane derivative is 4-tert.-butyl-4'-methoxydibenzoylmethane. The composition of the invention preferably comprises 0.1 to 10%, more preferably 0.2 to 5%, further more preferably 0.4 to 3%, by weight dibenzoylmethane or a derivative thereof based on total weight of the composition and including all ranges subsumed therein.

The composition preferably comprises a UV-B organic sunscreen selected from the class of cinnamic acid, salicylic acid, diphenyl acrylic acid and derivatives thereof. Illustrative non-limiting example of UV-B sunscreens which are commercially available and useful for inclusion in the composition of the invention are Octisalate™, Homosalate™, NeoHelipan™, Octocrylene™, Oxybenzone™ or Parsol MCX™. The UV-B sunscreen is most preferably 2-ethyl-hexyl-4-methoxy cinnamate which is commercially available as Parsol MCX. The UV-B organic sunscreen is preferably included in 0.1 to 10%, more preferably 0.1 to 7 % by weight of the composition. It has been observed that presence of an organic UV-B sunscreen like 2-ethyl-hexyl-4-methoxy cinnamate causes further rapid degradation of the UV-A dibenzoylmethane sunscreen in the presence of UV radiation. The presence of the rosmarinic acid ester compound is found to be very efficacious in stabilizing the composition even when UV-B sunscreens are present.

Useful inorganic sun-blocks are also preferably used in the present invention. These include, for example, zinc oxide, iron oxide, silica, such as fumed silica, and titanium dioxide.

Preservatives can also be added into the compositions to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatibility between the preservatives and other ingredients. Preservatives are preferably employed in amounts ranging from 0.01% to 2% by weight of the composition.

A variety of other optional materials may be formulated into the compositions. These may include: antimicrobials such as 2-hydroxy-4,2',4'-trichlorodiphenylether (triclosan), 2,6-dimethyl-4-hydroxychlorobenzene, and 3,4,4'-trichlorocarbanilide; scrub and exfoliating particles such as polyethylene and silica or alumina; cooling agents such as menthol; skin calming agents such as aloe vera; and colorants. In addition, the compositions may further include 0 to 10% by weight of opacifiers and pearlizers such as ethylene glycol distearate, titanium dioxide or Lytron® 621 (Styrene/Acrylate copolymer); all of which are useful in enhancing the appearance or properties of the product.

Diluents other than water can include liquid or solid emollients, solvents, humectants, thickeners and powders. Examples of each of these types of vehicle, which can be used singly or as mixtures of one or more vehicles, are as follows:

Emollients, such as stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate;;

Solvents, such as ethyl alcohol, isopropanol, acetone, ethylene glycol monoethyl ether, diethylene glycol monobutyl ether, diethylene glycol monoethyl ether;

In case of soap bars, it may contain particles that are greater than 50 μm in average diameter that help remove dry skin. Not being bound by theory, the degree of exfoliation depends on the size and morphology of the particles. Large and rough particles are usually very harsh and irritating. Very small particles may not serve as effective exfoliants. Such exfoliants used in the art include natural minerals such as silica, talc, calcite, pumice, tricalcium phosphate; seeds such as rice, apricot seeds, etc; crushed shells such as almond and walnut shells; oatmeal; polymers such as polyethylene and polypropylene beads, flower petals and leaves; microcrystalline wax beads; jojoba ester beads, and the like. These exfoliants come in a variety of particle sizes and morphology ranging from micron sized to a few mm. They also have a range of hardness. Some examples are talc, calcite, pumice, walnut shells, dolomite and polyethylene.

Advantageously, active agents other than skin conditioning agents defined above may be added to the composition. These active ingredients may be advantageously selected from bactericides, vitamins, anti-acne actives; anti-wrinkle, anti-skin atrophy and skin repair actives; skin barrier repair actives; non-steroidal cosmetic soothing actives; artificial tanning agents and accelerators; skin lightening actives; sunscreen actives; sebum stimulators; sebum inhibitors; anti-oxidants; protease inhibitors; skin tightening agents; anti-itch ingredients; hair growth inhibitors; 5-alpha reductase inhibitors; desquamating enzyme enhancers; anti-glycation agents; or mixtures thereof; and the like.

These active agents may be selected from water-soluble active agents, oil soluble active agents, pharmaceutically acceptable salts and mixtures thereof. The term "active agent" as used herein, means personal care actives which can be used to deliver a benefit to the skin and/or hair and which generally are not used to confer a skin conditioning benefit, such are delivered by emollients as defined above. The term "safe and effective amount" as used herein, means an amount of active agent high enough to modify the condition to be treated or to deliver the desired skin care benefit, but low enough to avoid serious side effects. The term "benefit," as used herein, means the therapeutic, prophylactic, and/or chronic benefits associated with treating a particular condition with one or more of the active agents described herein. What is a safe and effective amount of the active agent(s) will vary with the specific active agent, the ability of the active to penetrate through the skin, the age, health condition, and skin condition of the user, and other like factors.

Powders, such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The cosmetically acceptable base is usually from 10 to 99.9%, preferably from 50 to 99% by weight of the composition, and can, in the absence of other personal care adjuncts, form the balance of the composition.

The composition of the invention may comprise a conventional deodorant base as the cosmetically acceptable carrier. By a deodorant is meant a product in the stick, roll-on, or propellant medium which is used for personal deodorant benefit e.g. application in the underarm area which may or may not contain anti-perspirant actives.

Deodorant compositions can generally be in the form of firm solids, soft solids, gels, creams, and liquids and are dispensed using applicators appropriate to the physical characteristics of the composition. Deodorant compositions which are delivered through roll-ons generally comprise a liquid carrier. Such liquid carrier can be hydrophobic or comprise a mixture of both hydrophilic and hydrophobic liquids. They may be in the form of an emulsion or a microemulsion. The liquid carrier or mixture of carriers often constitutes from 30 to 95% by weight of the composition and in many instances from 40 to 80%. Hydrophobic liquid carriers commonly can comprise one or more materials selected within the chemical classes of siloxanes, hydrocarbons, branched aliphatic alcohols, esters and ethers that have a melting point not higher than 25°C and a boiling point of at least 100°C. Hydrophilic carrier liquids that can be employed in compositions herein commonly comprise water and/or a mono or polyhydric alcohol or water-miscible homologue. Monohydric alcohols often are short chain, by which is meant that they contain up to 6 carbons, and in practice is most often ethanol or sometimes iso-propanol. Polyhydric alcohols commonly comprise ethylene or propylene glycol, or a homologue can be employed such as diethylene glycol. Other than this suitable other vehicle and component used for deodorant composition can be added.

Preferably, when the composition is in the form of a hand sanitizer composition the cosmetically acceptable base may comprises of alcohol and water. The most preferred alcohols are ethyl alcohol and isopropyl alcohol. Even a mixture of two or more alcohol can preferably be used in the hand sanitizer composition. The amount of alcohol preferably in the range of 50 to 95%, more preferably 60 to 80% and most preferably 65 to 80% by weight of the hand sanitizer composition.

The compositions of the present invention can comprise a wide range of other optional components. The CTFA Personal care Ingredient Handbook, Second Edition, 1992, which is incorporated by reference herein in its entirety, describes a wide variety of non-limiting personal care and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, pH adjusters, natural extracts, essential oils, skin sensates, skin soothing agents, and skin healing agents.

The present invention also discloses a method of cleaning or disinfecting a surface comprising the steps of applying a composition according to the invention on to said surface in case of a leave-on composition. This method optionally comprises an additional step of at least partially removing the composition from the surface if it is in the form of a wash-off composition. Preferably, the step of at least partially removing the composition is carried out less than 5 minutes after the step of applying the composition on the substrate.

The present invention also discloses a use of a composition of the present invention as disclosed above for antimicrobial benefit. Therefore the composition of the present invention able to provide prolonged/long-lasting antimicrobial benefits. The preferred intended use/method of the composition of the present invention is non-therapeutic and/or cosmetic.

The present invention also discloses a use of a composition of the present invention as disclosed above for hand hygiene.

Now the invention will be demonstrated by the following non limiting example.

**Examples:**

The following protocol was used to evaluate biocidal activity. IN-VITRO TIME-KILL PROTOCOL - ASTM 2783

Preparation of the bacterial culture:

For these experiments, *Escherichia.coli* ATCC 10536 was used in the study, which represents gram-negative bacteria. The bacteria were grown overnight on Tryptic soya agar (TSA) plate. The bacterial cell density was then adjusted at 620 nm to a pre-calibrated optical density to get the final count of $10^9$ cfu/mL in saline (0.86% NaCl) by using a spectrophotometer.

Assay Protocol:

9.9 mL of the composition of different examples (as stated above) was taken in different sample containers to each of those container 0.1mL of bacterial culture was added just before performing the assay and mixed well to obtain a mixture. A timer was started immediately after the addition of the culture. The mixture was kept for a specific contact of 10 seconds and 30 seconds.

At the end of the each contact time (10 seconds and 30 seconds), the antibacterial activity of the samples was neutralized immediately, by addition of 1 mL each of the above mixture to 9 mL of an appropriate neutralizing broth which is validated for the test system. The neutralized samples were then serially diluted upto 5 dilution in neutralizer broth and plated on TSA (40gpL - Difco) in duplicates.

The log reduction was calculated by comparing with the bacterial control. The bacterial control used for this purpose was a mixture prepared by addition of 0.1 mL of bacterial culture to 9.9 mL of saline; the mixture was then serially diluted and plated on TSA. After solidification of the TSA plates, the plates were incubated at 37°C for 48 hours. The colonies on the plates were counted.

Log reduction greater than 5 means 99.99% reduction in the number of CFU and log reduction less than 0.5 means no reduction in the number of CFU that means no antimicrobial efficacy. Log reduction greater than 5 also denotes complete kill.

Effect of different salts:

The following samples were prepared for testing the effect of different salts in combination with the carboxylic acid with $pK_a$ greater than 4.5, on antimicrobial efficacy:

Example A: 2% ammonium chloride (obtained from Merck) solution was prepared in distilled water.

Example B: 2% ammonium citrate (obtained from Sigma) solution was prepared in distilled water.

Example C: 2% ammonium benzoate (obtained from Aldrich) solution was prepared in distilled water.

Example D: A solution of 2% potassium chloride (Obtained from SRL) and 0.2% octanoic acid was prepared in distilled water.

Example E: A solution of 2% sodium chloride (Obtained from SD Fine Chemical Ltd.) and 0.2% octanoic acid was prepared in distilled water.

Example F: 0.2% octanoic acid ($pK_a = 4.89$, obtained from Aldrich)) solution was prepared in distilled water.

Example G: 0.2% 4-methyl octanoic acid ($pK_a = 4.89$, obtained from Aldrich)) acid solution was prepared in distilled water.

Example 1: A solution of 2% ammonium chloride and 0.2% octanoic ($pK_a = 4.89$) acid was prepared in distilled water.

Example 2: A solution of 2% ammonium chloride and 0.2% 4-methyl octanoic acid ($pK_a = 4.89$) acid was prepared in distilled water.

Example 3: A solution of 2% ammonium citrate and 0.2% octanoic ($pK_a = 4.89$) acid was prepared in distilled water.

Example 4: A solution of 2% ammonium benzoate and 0.2% octanoic ($pK_a = 4.89$) acid was prepared in distilled water.

The pH of all the examples were adjusted to 4.9 ± 0.3 (close to skin pH).

All these samples were tasted for antimicrobial efficacy using the protocol described in previous section. The results are summarized below in Table 1.

Table 1

| Example No. | Log reduction | |
|---|---|---|
| | 10 seconds | 30 seconds |
| A | <0.5 | <0.5 |
| B | <0.5 | <0.5 |
| C | <0.5 | <0.5 |
| D | 1.1 ± 0.24 | 1.5 ± 0.26 |
| E | 1.0 ± 0.31 | 1.3 ± 0.33 |
| F | 0.6 ± 0.31 | 0.8 ± 0.26 |
| G | 0.5 ± 0.28 | 0.9 ± 0.37 |

(continued)

| Example No. | Log reduction | |
|---|---|---|
| | 10 seconds | 30 seconds |
| 1 | > 5 | > 5 |
| 2 | > 5 | > 5 |
| 3 | > 5 | > 5 |
| 4 | > 5 | > 5 |

From Table 1 it is evident that ammonium salt having at least one covalently bonded hydrogen along with a carboxylic acid with $pK_a$ more than 4.5 (Example 1 to 4) is providing much higher log reduction value (> 5) when compared with ammonium salt (Example A, B and C) and carboxylic acid (Example F and G) alone. It is also clear from the above table that non-ammonium salt in combination with carboxylic acid with $pK_a$ more than 4.5 (Example D and E) does not provide required antimicrobial benefit.

Effect of carboxylic acid with different $pK_a$ value:

The following samples were prepared for testing the effect of different carboxylic acid with different $pK_a$ values in combination with an anionic surfactant with ammonium group as counter ion, on antimicrobial efficacy:

Example H: 1% ALS (ammonium lauryl sulphate, obtained from Fluka) solution was prepared in distilled water.

Example I: A solution of 0.25% methyl caprylic acid ($pK_a$ = 4.93, obtained from Aldrich) was prepared in distilled water.

Example J: A solution of 0.25% octanoic acid ($pK_a$ = 4.89) was prepared in distilled water.

Example K: A solution of 0.5% cyclohexanoic acid ($pK_a$ = 4.91, obtained from Alrdich) was prepared in distilled water.

Example L: A solution of 1% ALS and 2% citric acid ($pK_a$ = 3.13, obtained from Sigma-Aldrich) was prepared in distilled water.

Example M: A solution of 1% ALS and 2% malic acid ($pK_a$ = 3.4, obtained from Sigma-Aldrich) was prepared in distilled water.

Example N: A solution of 1% ALS and 2% malonic acid ($pK_a$ = 2.82, obtained from Vetec) was prepared in distilled water.

Example O: A solution of 1% ALS and 2% glycolic acid ($pK_a$ = 3.6, obtained from Vetec) was prepared in distilled water.

Example P: A solution of 1% ALS and 2% benzoic acid ($pK_a$ = 4.2, obtained from Sigma-Aldrich) was prepared in distilled water.

Example Q: A solution of 1% ALS and 2% hydroxy benzoic acid ($pK_a$ = 4.3, obtained from Sigma-Aldrich) was prepared in distilled water.

Example R: A solution of 1% ALS and 2% of 4 - hydroxy benzoic acid ($pK_a$ = 4.5, obtained from Aldrich) was prepared in distilled water.

Example 5: A solution of 1% ALS and 0.25% methyl caprylic acid ($pK_a$ = 4.93) was prepared in distilled water.

Example 6: A solution of 1% ALS and 0.25% octanoic acid ($pK_a$ = 4.89) was prepared in distilled water.

Example 7: A solution of 1% ALS and 0.5% cyclohexanoic acid ($pK_a$ = 4.93) was prepared in distilled water.

The pH of all the examples were adjusted to 4.9 ± 0.3 (close to skin pH).

All these samples were tasted for antimicrobial efficacy using the protocol described in previous section. The results are summarized below in Table 2.

Table 2

| Example No. | Log reduction | |
|---|---|---|
| | 10 seconds | 30 seconds |
| H | 0.9 ± 0.14 | 1.8 ± 0.31 |
| I | < 0.5 | 0.7 ± 0.11 |
| J | < 0.5 | 0.9 ± 0.22 |
| K | < 0.5 | 0.8 ± 0.21 |
| L | < 0.5 | < 1 |
| M | < 0.5 | < 1 |
| N | < 0.5 | < 1 |
| O | < 0.5 | < 1 |
| P | 1.2 ± 0.14 | 3.2 ± 0.28 |
| Q | 0.9 ± 0.21 | 2.8 ± 0.19 |
| R | 1.3 ± 0.22 | 3.6 ± 0.24 |
| 5 | > 5 | > 5 |
| 6 | > 5 | > 5 |
| 7 | > 5 | > 5 |

From Table 2, it is evident that an anionic surfactant with ammonium group as counter ion in combination with a carboxylic acid with $pK_a$ more than 4.5 (Example 5 to 7) provides complete kill (log reduction >5) when compared with an anionic surfactant with ammonium group as counter ion (Example H) and carboxylic acid (Example I, J and K) alone. It is also clear from the above table that a carboxylic acid with $pK_a$ less than or equal to 4.5 (Example L to R) does not provide required antimicrobial benefit.

Effect of different anionic surfactant:

The following samples were prepared for testing the effect of different anionic surfactant in combination with the carboxylic acid with $pK_a$ greater than 4.5, on antimicrobial efficacy:

Example S: A solution of 7.5 % SLES (sodium laureth sulphate) and 1% octanoic acid was prepared in distilled water.

Example T: A solution of 7.5 % SLS (sodium lauryl sulphate) and 1% octanoic acid was prepared in distilled water.

Example 8: A solution of 7.5 % ALS and 1% octanoic acid was prepared in distilled water.

Example 9: A solution of 7.5 % ALS and 1% 4-methyl caprylic acid was prepared in distilled water.

Example 10: A solution of 7.5 % ALES (ammonium lauryl ether sulphate) and 1% methyl caprylic acid was prepared in distilled water.

Example 11: A solution of 7.5 % Marlinat (242/90M)* and 1% methyl caprylic acid was prepared in distilled water.

*MIPA (Monoisopropanolammonium) salt of lauryl ether sulfate, obtained from SASOL.
The pH of all the examples were adjusted to 4.9 ± 0.3 (close to skin pH).
All these samples were tasted for antimicrobial efficacy using the protocol described in previous section. The results are summarized below in Table 3.

Table 3

| Example No. | Log reduction | |
|---|---|---|
| | 10 seconds | 30 seconds |
| S | 0.14 ± 0.23 | 0.3 ± 0.11 |
| T | 1.4 ± 0.31 | 2.5 ± 0.21 |
| 8 | 4.19 ± 0.22 | > 5 |
| 9 | > 5 | > 5 |

(continued)

| Example No. | Log reduction | |
|---|---|---|
| | 10 seconds | 30 seconds |
| 10 | > 5 | > 5 |
| 11 | > 5 | > 5 |

From Table 3, it is evident that an anionic surfactant with ammonium group as counter ion in combination with a carboxylic acid with $pK_a$ more than 4.5 (Example 8 to 11) provides much higher log reduction value when compared with anionic surfactants with counter ion other than ammonium (Example S and T) in combination with a carboxylic acid with $pK_a$ more than 4.5.
Therefore from the above discussion and examples it is evident that it possible by way of present invention to provide a synergistic antimicrobial composition with ability to provide the antimicrobial benefit at relatively short contact time and at skin pH.

**Claims**

1. An antimicrobial composition comprising:

   a) 5 to 40% by weight of an ammonium salt having at least one covalently bonded hydrogen attached to the nitrogen or of an anionic surfactant with ammonium group as counter ion or mixtures thereof; and,
   b) 0.1 to 20% by weight of a carboxylic acid with $pK_a$ value is in between 4.5 to 6.5 and wherein the carboxylic acid is selected from fatty acid having chain length $C_4$ to $C_{16}$, Cyclic aliphatic carboxylic acid, aromatic carboxylic acid or mixtures thereof;
   wherein the pH of the composition is in the range of 4.5 to 6.5;
   and wherein the total amount of carboxylic acid with $pK_a$ value greater than 4.5 is from 0.1 to 20% by weight.

2. A composition as claimed in claim 1 wherein the ammonium salt is selected from ammonium chloride, ammonium benzoate, ammonium citrate, ammonium carbonate, ammonium acetate, ammonium sulphate, isopropyl ammonium chloride or mixtures thereof.

3. A composition as claimed in claim 1 wherein the surfactant with ammonium group as counter ion is selected from ammonium lauryl sulphate, ammonium laureth sulfate, Ammonium dodecyl benzene sulfonate, or a combination thereof.

4. A composition as claimed in any one of the preceding claims in the form of a bar, liquid or gel.

5. A composition as claimed in any one of the preceding claims wherein the composition is in the form of a leave-on composition.

6. A composition as claimed in any one of the preceding claims 1 to 4 wherein the composition is in the form of a wash-off composition.

7. A composition as claimed in claim 6 further comprising at least one surfactant.

8. A composition as claimed in claim 7 wherein the surfactant is other than soap.

9. A non-therapeutical method of cleaning or disinfecting a surface comprising the step of applying a composition as claimed in any one of the preceding claims on to said surface.

10. A non-therapeutical method as claimed in claim 9 wherein the composition is in the form of a wash-off composition and wherein there is an additional step of at least partially removing the applied composition.

11. A non-therapeutical method as claimed in claim 10 wherein the step of at least partially removing the composition is carried out in less than 5 minutes after the step of applying the composition on the substrate.

12. Non-therapeutical use of a composition as claimed in any one of the preceding claims 1 to 8 for antimicrobial benefit.

**Patentansprüche**

1. Antimikrobielle Zusammensetzung, umfassend:

a) 5 bis 40 Gewichts-% eines Ammoniumsalzes mit mindestens einem kovalent gebundenen Wasserstoff, verbunden mit dem Stickstoff, oder ein anionisches Tensid mit Ammoniumgruppe als Gegenion oder Mischungen davon; und
b) 0,1 bis 20 Gewichts-% einer Carbonsäure mit einem $pK_a$-Wert zwischen 4,5 und 6,5 und wobei die Carbonsäure unter einer Fettsäure einer Kettenlänge von $C_4$ bis $C_{16}$, cyclischer aliphatischer Carbonsäure, aromatischer Carbonsäure oder Mischungen davon ausgewählt ist,

wobei der pH-Wert der Zusammensetzung in dem Bereich von 4,5 bis 6,5 liegt und
wobei die Gesamtmenge der Carbonsäure mit einem $pK_a$-Wert von mehr als 4,5 0,1 bis 20 Gew.-% beträgt.

2. Zusammensetzung, wie im Anspruch 1 beansprucht, wobei das Ammoniumsalz unter Ammoniumchlorid, Ammoniumbenzoat, Ammoniumcitrat, Ammoniumcarbonat, Ammoniumacetat, Ammoniumsulfat, Isopropylammoniumchlorid oder Mischungen davon ausgewählt ist.

3. Zusammensetzung, wie im Anspruch 1 beansprucht, wobei das Tensid mit Ammoniumgruppe als Gegenion unter Ammoniumlaurylsulfat, Ammoniumlaurethsulfat, Ammoniumdodecylbenzolsulfonat oder einer Kombination davon ausgewählt ist.

4. Zusammensetzung, wie in irgendeinem der vorhergehenden Ansprüche beansprucht, in Form eines Stifts, einer Flüssigkeit oder eines Gels.

5. Zusammensetzung, wie in irgendeinem der vorhergehenden Ansprüche beansprucht, wobei die Zusammensetzung in Form einer Leave-on-Zusammensetzung vorliegt.

6. Zusammensetzung, wie in irgendeinem der vorhergehenden Ansprüche 1 bis 4 beansprucht, wobei die Zusammensetzung in Form einer Abwaschzusammensetzung vorliegt.

7. Zusammensetzung, wie im Anspruch 6 beansprucht, die ferner mindestens ein Tensid enthält.

8. Zusammensetzung, wie im Anspruch 7 beansprucht, wobei das Tensid, außer als Seife, vorliegt.

9. Nicht-therapeutisches Verfahren zum Reinigen und Desinfizieren einer Oberfläche, umfassend den Schritt des Aufbringens einer Zusammensetzung, wie in irgendeinem der vorhergehenden Ansprüche beansprucht, auf die Oberfläche.

10. Nicht-therapeutisches Verfahren, wie in Anspruch 9 beansprucht, wobei die Zusammensetzung in Form einer Abwaschzusammensetzung vorliegt und wobei darin ein zusätzlicher Schritt des zumindest teilweisen Entfernens der aufgetragenen Zusammensetzung vorgenommen wird.

11. Nicht-therapeutisches Verfahren, wie im Anspruch 10 beansprucht, wobei der Schritt des zumindest teilweisen Entfernens der Zusammensetzung innerhalb von weniger als 5 Minuten nach dem Schritt des Aufbringens der Zusammensetzung auf das Substrat durchgeführt wird.

12. Nicht-therapeutische Verwendung einer Zusammensetzung, wie in irgendeinem der vorhergehenden Ansprüche 1 bis 8 beansprucht, zum Erzielen eines antimikrobiellen Vorteils.

## Revendications

1. Composition antimicrobienne comprenant :

   a) de 5 à 40 % en masse d'un sel d'ammonium présentant au moins un hydrogène lié de manière covalente fixé à l'azote ou d'un tensioactif anionique avec un groupe ammonium comme contre-ion ou des mélanges de ceux-ci ; et,
   b) de 0,1 à 20 % en masse d'un acide carboxylique avec une valeur $pK_a$ de 4,5 à 6,5 et dans laquelle l'acide carboxylique est choisi parmi un acide gras ayant une longueur de chaîne $C_4$ à $C_{16}$, un acide carboxylique aliphatique cyclique, un acide carboxylique aromatique ou des mélanges de ceux-ci ;

   dans laquelle le pH de la composition se trouve dans l'intervalle de 4,5 à 6,5 ;
   et dans laquelle la quantité totale d'acide carboxylique avec une valeur de $pK_a$ supérieure à 4,5 est de 0,1 à 20 % en masse.

2. Composition selon la revendication 1, dans laquelle le sel d'ammonium est choisi parmi le chlorure d'ammonium, benzoate d'ammonium, citrate d'ammonium, carbonate d'ammonium, acétate d'ammonium, sulfate d'ammonium, chlorure d'isopropylammonium ou mélanges de ceux-ci.

3. Composition selon la revendication 1, dans laquelle le tensioactif avec un groupe ammonium comme contre-ion est choisi parmi le laurylsulfate d'ammonium, laureth sulfate d'ammonium, dodécylbenzène sulfonate d'ammonium, ou une combinaison de ceux-ci.

4. Composition selon l'une quelconque des revendications précédentes dans la forme d'une barre, d'un liquide ou d'un gel.

5. Composition selon l'une quelconque des revendications précédentes, dans laquelle la composition est dans la forme d'une composition sans rinçage.

6. Composition selon l'une quelconque des revendications 1 à 4 précédentes, dans laquelle la composition est dans la forme d'une composition à rincer.

7. Composition selon la revendication 6 comprenant de plus au moins un tensioactif.

8. Composition selon la revendication 7, dans laquelle le tensioactif est différent du savon.

9. Procédé non-thérapeutique de nettoyage ou désinfection d'une surface comprenant l'étape d'application d'une composition selon l'une quelconque des revendications précédentes sur ladite surface.

10. Procédé non-thérapeutique selon la revendication 9, dans lequel la composition est dans la forme d'une composition à rincer et dans laquelle il y a une étape supplémentaire d'élimination au moins partielle de la composition appliquée.

11. Procédé non-thérapeutique selon la revendication 10, dans lequel l'étape d'élimination au moins partielle de la composition est réalisée en moins de 5 minutes après l'étape d'application de la composition sur le substrat.

12. Utilisation non thérapeutique d'une composition selon l'une quelconque des revendications 1 à 8 précédentes pour un bénéfice anti-microbien.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008014247 A, Lu
- US 6051614 A
- US 3050467 B1, Horowitz
- US 2011224120 AA, Henkel
- WO 0128338 A

**Non-patent literature cited in the description**

- The CTFA Personal care Ingredient Handbook. 1992